# EUROPÄISCHE PATENTSCHRIFT

(11) **EP 1 695 701 B1**
(45) Veröffentlichungstag und Bekanntmachung des Hinweises auf die Patenterteilung: **07.01.2015**
(21) Anmeldenummer: 06115508.1
(22) Anmeldetag: 20.03.2003
(51) Int. Cl.: A61K 9/72, A61K 31/46, A61P 11/06

(54) **HFA-Suspensionsformulierungen enthaltend ein Anticholinergikum**
HFA suspension formulations comprising an anticholinergic agent
Formulations de suspension contenant un gaz propulseur HFA et un anticholinergique

(30) Priorität: 28.03.2002 DE 10214263
(43) Veröffentlichungstag der Anmeldung: 30.08.2006
(62) Teilanmeldung aus: 03745193.7
(73) Patentinhaber: Boehringer Ingelheim Pharma GmbH & Co. KG, 55216 Ingelheim am Rhein (DE)
(72) Erfinder: Schmelzer, Dr., Christel, 55218 Ingelheim (DE)
(74) Vertreter: Eckhardt, Conrad

(56) Entgegenhaltungen:
- WO-A-00/07567
- WO-A-01/78743
- WO-A-02/30928
- WO-A-02/36106
- WO-A-02/38154
- WO-A1-00/69468

## Beschreibung

Die Erfindung betrifft Druckgaszubereitungen für Dosieraerosole mit Suspensionsformulierungen des kristallinen Monohydrats von (1α,2β,4β,5α,7β)-7-[(hydroxydi-2-thienylacetyl)oxy]-9,9-dimethyl-3-oxa-9-azoniatricyclo[3.3.1.0^{2,4}]nonane-bromid, Verfahren zu dessen Herstellung, sowie dessen Verwendung zur Herstellung eines Arzneimittels, insbesondere zur Herstellung eines Arzneimittels mit anticholinerger Wirkung.

### Hintergrund der Erfindung

Die Verbindung (1α,2β,4β,5α,7β)-7-[(hydroxydi-2-thienylacetyl)oxy]-9,9-dimethyl-3-oxa-9-azoniatricyclo[3.3.1.0^{2,4}]nonane-bromid, ist aus der Europäischen Patentanmeldung EP 418 716 A1 bekannt und weist die folgende chemische Struktur auf:

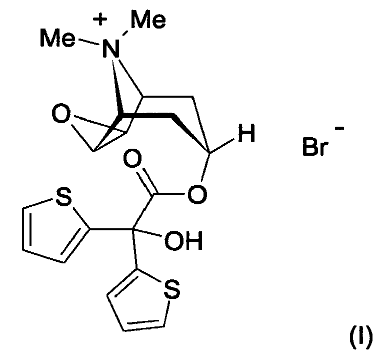

Die Verbindung besitzt wertvolle pharmakologische Eigenschaften und ist unter dem Namen Tiotropiumbromid (BA679) bekannt. Tiotropiumbromid stellt ein hochwirksames Anticholinergikum dar und kann deshalb bei der Therapie von Asthma oder COPD (chronic obstructive pulmonary disease = chronisch obstruktive Lungenerkrankung) einen therapeutischen Nutzen entfalten.

Die Applikation von Tiotropiumbromid erfolgt vorzugsweise auf inhalativem Wege.

Aufgabe der vorliegenden Erfindung ist die Bereitstellung von HFA-Dosieraerosolen enthaltend Tiotropiumbromid als den einzigen Wirkstoff in suspendierter Form.

### Detaillierte Beschreibung der Erfindung

Es wurde gefunden, daß Tiotropiumbromid je nach Wahl der Bedingungen, die bei der Reinigung des nach der technischen Herstellung erhaltenen Rohprodukts angewendet werden können, in unterschiedlichen kristallinen Modifikationen anfällt.

Es wurde gefunden, daß diese unterschiedlichen Modifikationen maßgeblich durch Wahl der zur Kristallisation eingesetzten Lösemittel sowie durch Wahl der beim Kristallisationsprozess gewählten Verfahrensbedingungen gezielt erhalten werden können. Für die Zwecke der Herstellung der erfindungsgemäßen Formulierungen hat sich das kristalline Tiotropiumbromid-monohydrat als besonders geeignet erwiesen.

Dementsprechend betrifft die vorliegende Erfindung Suspensionen des kristallinen Tiotropiumbromid-Monohydrats in den Treibgasen HFA 227 und/oder HFA 134a, gegebenenfalls in Mischung mit einem oder mehreren weiteren Treibgasen, vorzugsweise ausgewählt aus der Gruppe bestehend aus Propan, Butan, Pentan, Dimethylether, CHCIF₂, CH₂F₂, CF₃CH₃, Isobutan, Isopentan und Neopentan.

Erfindungsgemäß bevorzugt sind solche Suspensionen, die als Treibgas nur HFA 227, ein Gemisch aus HFA 227 und HFA 134a oder nur HFA 134a enthalten.
Wird in den erfindungsgemäßen Suspensionsformulierungen ein Gemisch der Treibgase HFA 227 und HFA 134a eingesetzt, so sind die Gewichtsverhältnisse, in denen diese beiden Treibgaskomponenten zum Einsatz gelangen können, frei variabel.
Werden in den erfindungsgemäßen Suspensionsformulierungen neben den Treibgasen HFA 227 und/oder HFA 134a ein oder mehrere weitere Treibgase, ausgewählt aus der Gruppe bestehend aus Propan, Butan, Pentan, Dimethylether, CHCIF₂, CH₂F₂, CF₃CH₃, Isobutan, Isopentan und Neopentan eingesetzt, so liegt der Anteil dieser weiteren Treibgaskomponente vorzugsweise unter 50 %, bevorzugt unter 40% besonders bevorzugt unter 30%.

Die erfindungsgemäßen Suspensionen enthalten vorzugsweise zwischen 0,001 bis 0,8% Tiotropium. Erfindungsgemäß bevorzugt sind Suspensionen, die 0,08 bis 0,5%, besonders bevorzugt 0,2 bis 0,4% Tiotropium enthalten.

Unter Tiotropium ist das freie Ammoniumkation zu verstehen. Die erfindungsgemäßen Treibgas- Suspensionen sind dadurch gekennzeichnet, daß sie Tiotropium in Form des durch seine für diese Anwendung herausragend geeigneten kristallinen Tiotropiumbromid-monohydrats enthalten. Dementsprechend betrifft die vorliegende Erfindung vorzugsweise Suspensionen, die zwischen 0,0012 und 1% kristallines Tiotropiumbromid-monohydrat enthalten. Erfindungsgemäß von besonderem Interesse sind Suspensionen, die 0,1 bis 0,62%, besonders bevorzugt 0,25 bis 0,5% kristallines Tiotropiumbromid-monohydrat enthalten.

Bei den im Rahmen der vorliegenden Erfindung genannten prozentualen Angaben, handelt es sich stets um Massenprozente. Werden Massenanteile für Tiotropium in Massenprozenten zum Ausdruck gebracht, sind die entsprechenden Werte für das im Rahmen der vorliegenden Erfindung bevorzugt zum Einsatz gelangende kristalline Tiotropiumbromid-monohydrat durch Multiplikation mit dem Umrechnungsfaktor 1,2495 erhältlich.

Gegebenenfalls wird im Rahmen der vorliegenden Erfindung statt des Begriffs Suspension auch der Begriff Suspensionsformulierung verwendet. Beide Begriffe sind im Rahmen der vorliegenden Erfindung als gleichbedeutend anzusehen.

Die erfindungsgemäßem treibgashaltigen Inhalationsaerosole bzw. Suspensionsformulierungen können ferner weitere Bestandteile wie oberflächenaktive Mittel (Tenside, Surfactants), Adjuvantien, Antioxidantien oder Geschmacksmittel enthalten.

Die in den erfindungsgemäßen Suspensionen gegebenenfalls enthaltenen oberflächenaktiven Mittel (Tenside, Surfactants) sind vorzugsweise ausgewählt aus der Gruppe bestehend aus Polysorbat 20, Polysorbat 80, Myvacet 9-45, Myvacet 9-08, Isopropylmyristat , Ölsäure, Propylenglycol, Polyethylenglycol, Brij, Ethyloleat, Glyceryltrioleat Glycerylmonolaurat , Glycerylmonooleat, Glycerylmonosterat, Glycerylmonoricinoleate, Cetylalcohol, Sterylalkohol, Cetylpyridinumchlorid, Blockpolymere, natürliches Öl, Ethanol und Isopropanol. Von den vorstehend genannten Suspensionshilfsstoffen gelangen bevorzugt zur Anwendung Polysorbat 20, Polysorbat 80, Myvacet 9-45, Myvacet 9-08 oder Isopropylmyristat. Besonders bevorzugt gelangen zur Anwendung Myvacet 9-45 oder Isopropylmyristat.
Sofern in den erfindungsgemäßen Suspensionen oberflächenaktive Mittel enthalten sind, werden diese vorzugsweise in einem Anteil von 0,0005 - 1 %, besonders bevorzugt 0,005 - 0,5 % eingesetzt.

Die in den erfindungsgemäßen Suspensionen gegebenenfalls enthaltenen Adjuvantien sind vorzugsweise ausgewählt aus der Gruppe bestehend aus Alanin, Albumin, Ascorbinsäure, Aspartam, Betain, Cystein, Phosphorsäure, Salpetersäure, Salzsäure, Schwefelsäure und Zitronensäure. Besonders bevorzugt gelangen dabei zur Anwendung Ascorbinsäure, Phosphorsäure, Salzsäure oder Zitronensäure, besonders bevorzugt Salzsäure oder Zitronensäure.
Sofern in den erfindungsgemäßen Suspensionen Adjuvantien enthalten sind, werden diese vorzugsweise in einem Anteil von 0,0001-1,0 %, bevorzugt 0,0005-0,1 %, besonders bevorzugt 0,001-0,01 % eingesetzt, wobei ein Anteil von 0,001-0,005 % erfindungsgemäß von besonderer Bedeutung ist.

Die in den erfindungsgemäßen Suspensionen gegebenenfalls enthaltenen Antioxidantien sind vorzugsweise ausgewählt aus der Gruppe bestehend aus Ascorbinsäure, Zitronensäure, Natriumedetat, Editinsäure, Tocopherolen, Butylhydroxytoluol, Butylhydroxyanisol und Ascorbylpalmitat, wobei Tocopherole, Butylhydroxytoluol, Butylhydroxyanisol oder Ascorbylpalmitat bevorzugt zum Einsatz gelangen.

Die in den erfindungsgemäßen Suspensionen gegebenenfalls enthaltenen Geschmacksmittel sind vorzugsweise ausgewählt aus der Gruppe bestehend aus Pefferminz, Sacharin, Dentomint, Aspartam und etherischen Ölen (beispielsweise Zimt, Anis, Menthol, Campher), wobei beispielsweise Pefferminz oder Dentomint® besonders bevorzugt sind.

Im Hinblick auf die inhalative Applikation ist es erforderlich, den Wirkstoff in feinteiliger Form bereitzustellen. Das wie im experimentellen Teil detailliert erläutert erhältliche kristalline Tiotropiumbromid-monohydrat wird dazu entweder gemahlen (mikronisiert) oder über andere technische, im Stand der Technik prinzipiell bekannte Verfahren (beispielsweise Präzipitation, Sprühtrocknung) in feinteiliger Form gewonnen.
Verfahren zur Mikronisierung von Wirkstoffen sind im Stand der Technik bekannt. Vorzugsweise weist der Wirkstoff nach Mikronisierung eine mittlere Teilchengröße von 0,5 bis 10µm, bevorzugt von 1 bis 6µm, besonders bevorzugt von 1,5 bis 5µm auf. Vorzugsweise weisen wenigstens 50%, bevorzugt wenigstens 60%, besonders bevorzugt wenigstens 70% der Wirkstoffteilchen eine Teilchengröße auf, die innerhalb der vorstehend genannten Größenbereiche liegt. Besonders bevorzugt liegen wenigstens 80%, höchst bevorzugt wenigstens 90% der Wirkstoffteilchen mit ihrer Partikelgröße in den vorstehend genannten Bereichen.

Überraschenderweise wurde gefunden, daß ebenfalls Suspensionen dargestellt werden können, die neben den genannten Treibgasen lediglich den Wirkstoff und keine weiteren Zusätze enthalten. Dementsprechnd zielt ein weiterer Aspekt der vorliegenden Erfindung auf Suspensionen, die lediglich den Wirkstoff ohne weitere Zusätze enthalten.

Zur Herstellung der erfindungsgemäßen Suspensionen kann nach im Stand der Technik bekannten Verfahren vorgegangen werden. Hierzu werden die Bestandteile der Formulierung mit dem oder den Treibgasen (ggf. bei niedrigen Temperaturen) gemischt und in geeignete Behälter abgefüllt.

Die vorstehend genannten erfindungsgemäßen treibgashaltigen Suspensionen können mittels im Stand der Technik bekannten Inhalatoren (pMDls = pressurized metered dose inhalers) appliziert werden. Dementsprechend betrifft ein weiterer Aspekt der vorliegenden Erfindung Arzneimittel in Form von wie vorstehend beschriebenen Suspensionen in Verbindung mit einem oder mehreren zur Verabreichung dieser Suspensionen geeigneten Inhalatoren. Ferner betrifft die vorliegende Erfindung Inhalatoren, dadurch gekennzeichnet, daß sie vorstehend beschriebene erfindungsgemäße treibgashaltige Suspensionen enthalten.
Die vorliegende Erfindung betrifft ferner Behälter (z.B. Kartuschen), die ausgestattet mit einem geeigneten Ventil in einem geeigneten Inhalator zur Anwendung gelangen können und die eine der vorstehend genannten erfindungsgemäßen treibgashaltigen Suspensionen enthalten. Geeignete Behälter (z.B. Kartuschen) und Verfahren zur Abfüllung dieser Kartuschen mit den erfindungsgemäßen treibgashaltigen Suspensionen sind aus dem Stand der Technik bekannt.

Aufgrund der pharmazeutischen Wirksamkeit von Tiotropium betrifft die vorliegende Erfindung ferner die Verwendung der erfindungsgemäßen Suspensionen zur Herstellung eines inhalativ oder nasal applizierbaren Arzneimittels, bevorzugt zur Herstellung eines Arzneimittels zur inhalativen oder nasalen Behandlung von Erkrankungen, in denen Anticholinergika einen therapeutischen Nutzen entfalten können.

Besonders bevorzugt betrifft die vorliegende Erfindung ferner die Verwendung der erfindungsgemäßen Suspensionen zur Herstellung eines Arzneimittels zur inhalativen Behandlung von Atemwegserkrankungen, bevorzugt von Asthma oder COPD.

Die nachfolgenden Beispiele dienen der exemplarischen, weitergehenden Illustration der vorliegenden Erfindung, ohne selbige auf deren Inhalt zu beschränken.

### Ausgangsmaterialien

### Kristallines Tiotropiumbromid-monohydrat:

Zur Darstellung des kristallinen Tiotropiumbromid-monohydrats kann das gemäß der EP 418 716 A1 erhältliche Tiotropiumbromid verwendet werden. Dieses wird dann wie nachfolgend beschrieben umgesetzt.

In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet.
Ausbeute : 13,4 kg Tiotropiumbromidmonohydrat (86 % d. Th.)

Das gemäß vorstehend beschriebener Vorgehensweise erhältliche kristalline Tiotropiumbromid-monohydrat wurde einer Untersuchung mittels DSC (Differential Scanning Calorimetry) unterworfen. Das DSC- Diagramm weist zwei charakteristische Signale auf. Das erste, relativ breite, endotherme Signal zwischen 50-120°C ist auf die Entwässerung des Tiotropiumbomid-monohydrats zur wasserfreien Form zurückzuführen. Das zweite, relativ scharfe, endotherme Maximum bei 230 ± 5°C ist dem Schmelzen der Substanz zuzuordnen. Diese Daten wurden mittels eines Mettler DSC 821 erhalten und mit dem Mettler Software-Paket STAR ausgewertet. Die Daten wurden bei einer Heizrate von 10 K/min erhoben.

Das kristalline Tiotropiumbromid-monohydrat wurde mittels IR-Spektroskopie charakterisiert. Die Daten wurden mittels eines Nicolet FTIR Spektrometers erhoben und mit dem Nicolet Softwarepaket OMNIC, Version 3.1 ausgewertet. Die Messung wurde mit 2,5µmol Tiotropiumbromid-monohydrat in 300 mg KBr durchgeführt.

Die nachstehende Tabelle faßt einige der wesentlichen Banden des IR-Spektrums zusammen.

| Wellenzahl (cm⁻¹) | Zuordnung | Schwingungstyp |
|---|---|---|
| 3570, 3410 | O-H | Streckschwingung |
| 3105 | Aryl C-H | Streckschwingung |
| 1730 | C=O | Streckschwingung |
| 1260 | Epoxid C-O | Streckschwingung |
| 1035 | Ester C-OC | Streckschwingung |
| 720 | Thiophen | Ringschwingung |

Das nach vorstehendem Verfahren erhältliche kristalline Tiotropiumbromidmonohydrat weist gemäß Einkristallröntgenstrukturanalyse eine einfache monoklinische Zelle mit folgenden Dimensionen auf: a = 18.0774 Å, b = 11.9711 Å, c = 9.9321 Å, β = 102.691°, V = 2096.96 Å³. Diese Daten wurden mittels eines AFC7R- 4-Kreisdiffraktometer (Rigaku) unter Verwendung von monochromatisierter Kupfer Kα-Strahlung erhoben. Die Strukturlösung und Verfeinerung der Kristallstruktur erfolgte mittels direkter Methoden (Programm SHELXS86) und FMLQ-Vefeinerung (Programm TeXsan).

Zur Darstellung der erfindungsgemäßen Suspensionen wird das nach vorstehendem Verfahren erhältliche kristalline Tiotropiumbromid-monohydrat nach an sich im Stand der Technik bekannten Verfahren mikronisiert, um den Wirkstoff in Form der mittleren Teilchengröße bereitzustellen, die den erfindungsgemäßen Spezifikationen entspricht.

Nachfolgend wird beschrieben, wie die Bestimmung der mittleren Teilchengröße des Wirkstoffs erfolgen kann.

### Partikelgrößenbestimmung von Tiotropiumbromidmonohydrat, mikronisiert:

### Meßgerät und Einstellungen:

Die Bedienung der Geräte erfolgte in Übereinstimmung mit den Bedienungsanleitungen des Herstellers.

| | |
|---|---|
| Meßgerät: | Laser-Beugungs-Spektrometer (HELOS), Sympatec |
| Dispergiereinheit: | Trockendispergierer RODOS mit Saugtrichter,Sympatec |
| Probenmenge: | 50 mg - 400 mg |
| Produktzufuhr: | Schwingrinne Vibri, Fa. Sympatec |
| Frequenz d. Vibrationsrinne: | 40 bis 100 % ansteigend |
| Dauer der Probenzufuhr: | 15 bis 25 sek. (im Fall von 200 mg) |
| Brennweite: | 100 mm (Meßbereich: 0,9 - 175 µm) |
| Meßzeit: | ca. 15 s (im Fall von 200 mg) |
| Zykluszeit: | 20 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

### Probenvorbereitung / Produktzufuhr:

ca. 200 mg der Prüfsubstanz werden auf einem Kartenblatt eingewogen.
Mit einem weiteren Kartenblatt werden alle größeren Agglomerate zerstoßen.
Das Pulver wird dann auf der vorderen Hälfte der Schwingrinne (ab ca. 1 cm vom vorderen Rand) fein verteilt aufgestreut.
Nach dem Start der Messung wird die Frequenz der Schwingrinne von ca. 40 % bis 100 % (gegen Ende der Messung) variiert. Die Zufuhr der Probe soll möglicht kontinuierlich sein. Die Produktmenge darf aber auch nicht zu groß sein, damit eine ausreichende Dispergierung erreicht wird. Die Zeit, in der jeweils die gesamte Probe zugeführt wird beträgt für 200 mg z. B. ca. 15 bis 25 sek.

### Formulierungsbeispiele

Suspensionen enthaltend neben Wirkstoff und Treibgas weitere Bestandteile:

| | | |
|---|---|---|
| a) | 0,02 % | Tiotropium* |
| | 0,20 % | Polysorbat 20 |
| | 99,78 % | HFA 227 |
| b) | 0,02 % | Tiotropium* |
| | 1,00 % | Isopropylmyristat |
| | 98, 98 % | HFA 227 |
| c) | 0,02 % | Tiotropium* |
| | 0,3 % | Myvacet 9-45 |
| | 99,68 % | HFA 227 |
| d) | 0,04 % | Tiotropium* |
| | 1 ,00 % | Myvacet 9-08 |
| | 98,96 % | HFA 227 |
| e) | 0,04 % | Tiotropium* |
| | 0,04 % | Polysorbat 80 |
| | 99,92 % | HFA 227 |
| f) | 0,04 % | Tiotropium* |
| | 0,005 % | Ölsäure |
| | 99,955 % | HFA 227 |
| g) | 0,02 % | Tiotropium* |
| | 0,1 % | Myvacet 9-45 |
| | 60,00 % | HFA 227 |
| | 39,88 % | HFA 134a |
| h) | 0,02 % | Tiotropium* |
| | 0,30 % | Isopropylmyristat |
| | 20,00 % | HFA 227 |
| | 79,68 % | HFA 134a |
| i) | 0,02 % | Tiotropium* |
| | 0,01 % | Ölsäure |
| | 60,00 % | HFA 227 |
| | 39,97 % | HFA 134a |

| | | |
|---|---|---|
| *eingesetzt in Form des Tiotropiumbromid-monohydrats ( Umrechnungsfaktor 1,2495) | | |

Suspensionen enthaltend lediglich Wirkstoff und Treibgas:

| | | |
|---|---|---|
| j) | 0,02 % | Tiotropium* |
| | 99,98 % | HFA 227 |
| k) | 0,02 % | Tiotropium* |
| | 99,98 % | HFA 134a |
| I) | 0,04 % | Tiotropium* |
| | 99,96 % | HFA 227 |
| m) | 0,04 % | Tiotropium* |
| | 99,96 % | HFA 134a |
| n) | 0,02 % | Tiotropium* |
| | 20,00 % | HFA 227 |
| | 79,98 % | HFA 134a |
| o) | 0,02 % | Tiotropium* |
| | 60,00 % | HFA 227 |
| | 39,98 % | HFA 134a |
| p) | 0,04 % | Tiotropium* |
| | 40,00 % | HFA 227 |
| | 59,96 % | HFA 134a |
| q) | 0,04 % | Tiotropium* |
| | 80,00 % | HFA 227 |
| | 19,96 % | HFA 134a |

| | | |
|---|---|---|
| *eingesetzt in Form des Tiotropiumbromid-monohydrats (Umrechnungsfaktor 1,2495) | | |

## Patentansprüche

1. Suspensionen des kristallinen Tiotropiumbromid-Monohydrats in den Treibgasen HFA 227 und/oder HFA 134a, gegebenenfalls in Mischung mit einem oder mehreren weiteren Treibgasen ausgewählt aus der Gruppe bestehend aus Propan, Butan, Pentan, Dimethylether, CHCIF₂, CH₂F₂, CF₃CH₃, Isobutan, Isopentan und Neopentan.

2. Suspensionen nach Anspruch 1, **dadurch gekennzeichnet, daß** sie zwischen 0,001 und 0,8% Tiotropium enthalten.

3. Suspensionen nach Anspruch 1 oder 2, **dadurch gekennzeichnet, daß** sie als weitere Bestandteile oberflächenaktive Mittel (Tenside, Surfactants), Adjuvantien, Antioxidantien und/oder Geschmacksmittel enthalten.

4. Suspensionen nach Anspruch 3, **dadurch gekennzeichnet, daß** sie als oberflächenaktive Mittel (Tenside, Surfactants) eine oder mehrere Verbindungen ausgewählt aus der Gruppe bestehend aus Polysorbat 20, Polysorbat 80, Myvacet 9-45, Myvacet 9-08, Isopropylmyristat, Ölsäure, Propylenglycol, Polyethylenglycol, Brij, Ethyloleat, Glyceryltrioleat Glycerylmonolaurat , Glycerylmonooleat, Glycerylmonosterat, Glycerylmonoricinoleate, Cetylalcohol, Sterylalkohol, Cetylpyridinumchlorid, Blockpolymere, natürliches Öl, Ethanol und Isopropanol enthalten.

5. Suspensionen nach Anspruch 3, **dadurch gekennzeichnet, daß** sie als Adjuvantien eine oder mehrere Verbindungen ausgewählt aus der Gruppe bestehend aus Alanin, Albumin, Ascorbinsäure, Aspartam, Betain, Cystein, Phosphorsäure, Salpetersäure, Salzsäure, Schwefelsäure und Zitronensäure enthalten.

6. Suspensionen nach Anspruch 3, **dadurch gekennzeichnet, daß** sie als Antioxidantien eine oder mehrere Verbindungen ausgewählt aus der Gruppe bestehend aus Ascorbinsäure, Zitronensäure, Natriumedetat, Editinsäure, Tocopherolen, Butylhydroxytoluol, Butylhydroxyanisol und Ascorbylpalmitat enthalten.

7. Suspensionen nach Anspruch 1 oder 2, **dadurch gekennzeichnet, daß** sie neben dem Wirkstoff und dem oder den Treibgasen keine weiteren Bestandteile enthalten.

8. Verwendung einer Suspension gemäß einem der Ansprüche 1 bis 7 zur Herstellung eines Arzneimittels, bevorzugt zur Herstellung eines Arzneimittels zur inhalativen oder nasalen Behandlung von Erkrankungen, in denen Anticholinergika einen therapeutischen Nutzen entfalten können.

9. Verwendung nach Anspruch 8, **dadurch gekennzeichnet, daß** es sich bei den Erkrankungen um Atemwegserkrankungen, bevorzugt um Asthma oder COPD handelt.

## Claims

1. Suspensions of crystalline tiotropium bromide monohydrate in the propellant gases HFA 227 and/or HFA 134a, optionally mixed with one or more further propellant gases selected from the group consisting of propane, butane, pentane, dimethyl ether, CHClF₂, CH₂F₂, CF₃CH₃, isobutane, isopentane and neopentane.

2. Suspensions according to claim 1, **characterised in that** they contain between 0.001 and 0.8% tiotropium.

3. Suspensions according to claim 1 or 2, **characterised in that** they contain as further constituents surface-active agents (surfactants), adjuvants, anti-oxidants and/or flavourings.

4. Suspensions according to claim 3, **characterised in that** they contain as surface-active agents (surfactants) one or more compounds selected from the group consisting of Polysorbate 20, Polysorbate 80, Myvacet 9-45, Myvacet 9-08, isopropyl myristate, oleic acid, propylene glycol, polyethylene glycol, Brij, ethyl oleate, glyceryl trioleate, glyceryl monolaurate, glyceryl monooleate, glyceryl monosterate, glyceryl monoricinoleates, cetyl alcohol, steryl alcohol, cetyl pyridinium chloride, block polymers, natural oil, ethanol and isopropanol.

5. Suspensions according to claim 3, **characterised in that** they contain as adjuvants one or more compounds selected from the group consisting of alanine, albumin, ascorbic acid, aspartame, betaine, cysteine, phosphoric acid, nitric acid, hydrochloric acid, sulphuric acid and citric acid.

6. Suspensions according to claim 3, **characterised in that** they contain as anti-oxidants one or more compounds selected from the group consisting of ascorbic acid, citric acid, sodium edetate, editic acid, tocopherols, butylhydroxytoluene, butylhydroxyanisole and ascorbyl palmitate.

7. Suspensions according to claim 1 or 2, **characterised in that** they contain no further constituents in addition to the active ingredient and the propellant gas or gases.

8. Use of a suspension according to one of claims 1 to 7 for producing a medicament, preferably for producing a medicament for inhalational or nasal treatment of diseases, in which anti-cholinergic agents may exhibit a therapeutic benefit.

9. Use according to claim 8, **characterised in that** the diseases are respiratory tract diseases, preferably asthma or COPD.

## Revendications

1. Suspensions de bromure de tiotropium monohydraté cristallin dans les gaz propulseurs HFA 227 et/ou HFA 134a, éventuellement en mélange avec un ou plusieurs autres gaz propulseurs sélectionnés dans le groupe constitué par le propane, le butane, le pentane, le diméthyléther, CHClF₂, CH₂F₂, CF₃CH₃, l'isobutane, l'isopentane et le néopentane.

2. Suspensions selon la revendication 1, **caractérisées en ce qu'**elles contiennent entre 0,001 et 0,8 % de tiotropium.

3. Suspensions selon la revendication 1 ou la revendication 2, **caractérisées en ce qu'**elles contiennent comme autres composants des agents modifiant la tension superficielle (tensioactifs, surfactants), des adjuvants, des antioxydants et/ou des agents aromatiques.

4. Suspensions selon la revendication 3, **caractérisées en ce qu'**elles contiennent comme agents modifiant la tension superficielle (tensioactifs, surfactants) un ou plusieurs composés sélectionnés dans le groupe constitué par Polysorbate 20, Polysorbate 80, Myvacet 9-45, Myvacet 9-08, le myristate d'isopropyle, l'acide oléique, le propylène glycol, le polyéthylène glycol, Brij, l'oléate d'éthyle, le trioléate de glycéryle, le monolaurate de glycéryle, le monooléate de glycéryle, le monostéarate de glycéryle, les monoricinoléates de glycéryle, l'alcool cétylique, l'alcool stéarylique, le chlorure de cétylpyridinum, les polymères à blocs, l'huile naturelle, l'éthanol et l'isopropanol.

5. Suspensions selon la revendication 3, **caractérisées en ce qu'**elles contiennent comme adjuvants un ou plusieurs composés sélectionnés dans le groupe constitué par l'alanine, l'albumine, l'acide ascorbique, l'aspartame, la bétaïne, la cystéine, l'acide phosphorique, l'acide nitrique, l'acide chlorhydrique, l'acide sulfurique et l'acide citrique.

6. Suspensions selon la revendication 3, **caractérisées en ce qu'**elles contiennent comme antioxydants un ou plusieurs composés sélectionnés dans le groupe constitué par l'acide ascorbique, l'acide citrique, l'édétate de sodium, l'acide édétique, les tocophérols, l'hydroxytoluène butylé, l'hydroxyanisole butylé et le palmitate d'ascorbyle.

7. Suspensions selon la revendication 1 ou la revendication 2, **caractérisées en ce qu'**elles ne comprennent pas d'autres composants que la substance active et le ou les gaz propulseurs.

8. Utilisation d'une suspension selon l'une des revendications 1 à 7 pour la préparation d'un médicament, préférentiellement pour la préparation d'un médicament destiné au traitement par inhalation ou par voie nasale de pathologies dans lesquelles les anticholinergiques peuvent déployer un effet thérapeutique.

9. Utilisation selon la revendication 8, **caractérisée en ce que** les pathologies sont des affections des voies respiratoires, préférentiellement l'asthme ou la BPCO.
